# EUROPEAN PATENT SPECIFICATION

(11) **EP 4 301 286 B1**
(45) Date of publication and mention of the grant of the patent: **11.02.2026**
(21) Application number: 22764203.0
(22) Date of filing: 04.03.2022
(51) Int. Cl.: A61F 2/44, A61F 2/30, B33Y 80/00

(54) **STANDALONE STABLE INTERBODY IMPLANT**
EIGENSTÄNDIGER STABILER ZWISCHENKÖRPER
IMPLANT INTERSOMATIQUE AUTONOME STABLE

(30) Priority: 04.03.2021 US 202163156825 P
(43) Date of publication of application: 10.01.2024
(73) Proprietor: Nexus Spine, LLC, Draper, Utah 84020 (US)
(72) Inventor: HALVERSON, Peter A., Draper, Utah 84020 (US); HAWKES, David T., Draper, Utah 84020 (US)
(74) Representative: Dummett Copp LLP
(86) International application number: PCT/US2022/019044
(87) International publication number: WO 2022/187718

(56) References cited:
- US-A1- 2008 161 927
- US-A1- 2010 152 856
- US-A1- 2010 152 856
- US-A1- 2015 328 005
- US-A1- 2015 328 005
- US-A1- 2017 172 755
- US-A1- 2018 303 623
- US-A1- 2019 274 841
- US-A1- 2019 336 305
- US-A1- 2019 343 644

## Description

### TECHNICAL FIELD

The present invention relates to interbody implants, and more particularly to spinal implants such as cervical spinal implants.

### BACKGROUND ART

The human spinal column allows the body to stand upright, sit, and bend. When the spinal column becomes disordered due to injury or disease, pain in the back or neck can occur. Treatment of spinal disorders often involves the use of one or more spinal implants to stabilize or otherwise treat the spine. Some implants and treatments seek to fuse the spine at one or more levels, eliminating motion between vertebrae as a mechanism for treating pain. Elimination of motion at a disordered level of the spine can reduce pain due to pinched nerves and the like at such a level. While such treatments have their place, the elimination of motion at one or more levels of the spine typically results in increased stresses at levels above and/or below the fused spinal levels. This often leads to spinal disorders at these additional levels, resulting in eventual recurrence of pain. Younger patients especially, can thus often expect to require further surgery at additional levels of the spine.

Spinal fusion implants are provided as several types, including interbody implants intended for implantation between adjacent vertebral bodies (in the space typically occupied by spinal discs in healthy spines) and pedicle rods extending between pedicles of adjacent vertebra. Such devices are often used together to maximally stabilize the spinal column at the desired level.

To prevent problems inherent to fusion implants, certain spinal implants seek to maintain or restore normal spinal mobility at a disordered spinal level instead of fusion of the disordered spinal level. One type of such implant is an interbody implant that is placed between adjacent vertebral bodies, in the space typically occupied by spinal discs in healthy spines. Such devices seek to replicate some or all of the functions of the spinal discs and function without the pedicle rods of spinal fusion devices. Accordingly, such devices can be known as standalone interbody implants.

Even though standalone interbody implants seek to maintain motion between adjacent vertebra, the intention is for all motion to occur within the implant itself. Unfortunately, most standalone interbody implants exhibit motion between the implant and the bone at the bone-to-implant interface. This motion has been observed in implants up to one year post implantation. Because this motion exists between the implant and the bone, the motion can lead to pain, fibrous tissue formation, and pseudoarthrosis. Accordingly, better standalone interbody implants are needed.

US 2015/328005 discloses a standalone interbody fusion device for engagement between adjacent vertebrae. The standalone interbody fusion device includes a spacer, and one or more inserts coupled to the spacer. The inserts provide apertures which are designed to retain bone fasteners, such as screws, and secure the implant to the adjacent vertebrae.

US 2010/152856 discloses a flexible element that for use as an intervertebral implant for insertion into an intervertebral disc space between adjacent vertebral bodies. The flexible element includes one or more struts extending from superior and inferior endplates and bending towards one another for connection with one or more internal beams.

### BRIEF SUMMARY OF THE INVENTION

The invention is set out in the appended set of claims.

Implementation of the invention provides standalone interbody implants designed to address the deficiencies of prior devices. Implementations of the invention provide standalone interbody implants limiting or eliminating motion between the implant and the bone at the bone-to-implant interface. Movement between vertebra is instead located intra-implant, thereby allowing the implant to preserve motion of the spinal level while providing stability all without compromising the bone-implant interface. This allows the implant to be secured to the bone while the bone then grows into and/or onto the implant. Accordingly, implementations of the invention address the deficiencies of the prior art.

Implementation of the invention provides implants including one or more pieces that are designed to replace joint motion from the implant-bone interface to an interface within the implant. Because the motion occurs within the implant instead of at the implant-bone interface, the implants relieve pain, reduce fibrous tissue formation, and alleviate pseudoarthrosis.

Implants according to implementations of the invention can have tailored stiffnesses by modifying the geometry of the contact surfaces within the implant and or by modifying flexures of the implant. Modification of the flexures may involve modification of any one or more of width, height, curvature, or length of the flexures.

Primary fixation of the implant to the bone using screws, blades, or other fixation devices decreases motion at the bone-implant interface. The flexibility of the implant itself decreases motion between the bone and the implant at the bone-implant interface. Additionally, porous and/or roughened surfaces and/or volumes of the implant facilitate boney attachment of the bone to the implant, further reducing the likelihood of movement at the bone-implant interface.

In some implementations, the use of three-dimensional (3D) printing to form the implant or portions thereof minimizes the number of parts needed to assemble the implant. Additionally, the use of 3D printing permits the creation of geometries that may not be available with traditional machining techniques. Some embodiments are formed as a single-piece implant. In other embodiments, the implant is manufactured in two or more pieces that are assembled using sliding or pressing assembly. The use of multiple-piece implants of some embodiments facilitates certain advantages such as decreasing the contact gap, improving the surface finish, machinability, or the like.

3D printing, while providing certain advantages, suffers from minimum distance requirements between objects or portions of the object, otherwise the features will fuse together. Traditional machining also has kerf limitations. Multi-piece manufacturing approaches allows for a desired performance of the implant while avoiding some of these limitations. Multi-piece construction also better allows for individualized control of other features such as surface finish, coatings, or variable-material compositions of the implant. One example of a variable-material composition is one material at the surface for promoting bone on-growth or ingrowth, and one material interior to the implant for intra-implant motion (e.g., rolling motion).

Flexure curvature and thickness can be altered in some implementations in controlled ways by inserting contact surfaces. By way of example, an insert can be shaped to induce a specific stress in one or more flexures to alter kinematics that would not otherwise be possible. Additionally, implant inserts can be mixed and matched in some implementations to create patient-specific implants.

According to implementations of the invention, an interbody implant is configured to permit intra-implant movement while minimizing movement at a bone-implant interface, and includes an implant body formed to permit a desired range of intra-implant movement between an upper surface thereof and a lower surface thereof, an upper fixation element adapted to secure an upper portion of the implant body to a superior bone, and a lower fixation element adapted to secure a lower portion of the implant body to an inferior bone.

According to some implementations, the implant body is formed as a single piece. According to some implementations, the implant is formed as a plurality of pieces assembled together. According to some implementations, one or more of the pieces assembled together includes a piece formed from three-dimensional (3D) printing. According to some implementations, one or more of the pieces assembled together includes a piece formed using a machining process. According to some implementations, the upper fixation element includes a first bone screw passing through an upper aperture of the implant body, and wherein the lower fixation element includes a second bone screw passing through a lower aperture of the implant body.

According to some implementations, each fixation element includes an element such as a screw or a blade. According to some implementations, an upper surface of the implant body and a lower surface of the implant body include roughened or porous surfaces to facilitate bone on-growth or ingrowth. According to some implementations, the upper portion of the implant body is flexibly affixed to the lower portion of the implant via a plurality of flexures extending between the upper portion and the lower portion.

According to some implementations, the implant body includes the upper portion of the implant body, the lower portion of the implant body, a plurality of flexures extending between the upper portion of the implant body and the lower portion of the implant body, wherein the upper portion, the lower portion, and the plurality of flexures are formed as a unitary construct, an upper porous insert formed to promote bone ongrowth or ingrowth affixed to the upper portion, a lower porous insert formed to promote bone ongrowth or ingrowth affixed to the lower portion, and an insert inserted between the upper portion of the implant body and the lower portion of the implant body to modify a kinematic profile between the upper portion of the implant body and the lower portion of the implant body imparted by the plurality of flexures in the absence of the insert.

According to some implementations, an interbody implant is configured to permit intra-implant movement while minimizing movement at a bone-implant interface and includes an implant body including a superior portion connected to an inferior portion by a plurality of flexures formed to permit a desired range of intra-implant movement between a superior surface thereof and an inferior surface thereof, a superior fixation element adapted to secure the superior portion of the implant body to a superior bone, and an inferior fixation element adapted to secure the inferior portion of the implant body to an inferior bone.

According to some implementations, the implant is formed as a plurality of pieces assembled together using an assembly method selected from the group consisting of a sliding assembly method and a press-fit assembly method. According to some implementations, one or more of the pieces assembled together includes a piece formed from three-dimensional (3D) printing. According to some implementations, one or more of the pieces assembled together includes a piece formed using a machining process.

According to some implementations, the superior fixation element includes a first bone screw passing through a superior aperture of the implant body, and wherein the inferior fixation element includes a second bone screw passing through an inferior aperture of the implant body. According to some implementations, each fixation element includes an element selected from the group consisting of a screw and a blade.

According to some implementations, a superior surface of the implant body and an inferior surface of the implant body include roughened or porous surfaces to facilitate bone on-growth or ingrowth. According to some implementations, the superior portion of the implant body is flexibly affixed to the inferior portion of the implant via a plurality of flexures extending between the superior portion and the inferior portion.

According to some implementations, the implant body includes the superior portion of the implant body, the inferior portion of the implant body, a plurality of flexures extending between the superior portion of the implant body and the inferior portion of the implant body, wherein the superior portion, the inferior portion, and the plurality of flexures are formed as a unitary construct, a superior porous insert formed to promote bone ongrowth or ingrowth affixed to the superior portion, an inferior porous insert formed to promote bone ongrowth or ingrowth affixed to the inferior portion, and an insert inserted between the superior portion of the implant body and the inferior portion of the implant body to modify a kinematic profile between the superior portion of the implant body and the inferior portion of the implant body imparted by the plurality of flexures in the absence of the insert.

According to some implementations, the plurality of flexures are modifiable to modify a stiffness of the implant body via modifications of the flexures including a modification selected from the group consisting of a width of the flexure, a height of the flexure, a curvature of the flexure, a thickness of the flexure, and a length of the flexure.

### BRIEF DESCRIPTION OF THE DRAWINGS

The objects and features of the present invention will become more fully apparent from the following description and appended claims, taken in conjunction with the accompanying drawings. Understanding that these drawings depict only typical embodiments of the invention and are, therefore, not to be considered limiting of its scope, the invention will be described and explained with additional specificity and detail through the use of the accompanying drawings in which:
Figure 1 provides a perspective view of an embodiment of a standalone interbody implant;
Figure 2 provides a side perspective view of an embodiment of a standalone interbody implant;
Figure 3 provides a side perspective view of an embodiment of a standalone interbody implant without fixation devices inserted therein;
Figure 4 provides a side perspective view of an embodiment of a standalone interbody implant illustrating one way in which inserts may be inserted into an implant body; and
Figure 5 provides a perspective view of an embodiment of a standalone interbody implant body prepared for reception of inserts therein.

### DETAILED DESCRIPTION OF THE INVENTION

The present invention is defined in claim 1 while preferred embodiments are set forth in the dependent claims.

A description of embodiments of the present invention will now be given with reference to the Figures. It is expected that the present invention may take many other forms and shapes, hence the following disclosure is intended to be illustrative and not limiting, and the scope of the invention should be determined by reference to the appended claims.

Embodiments of the invention provide standalone interbody implants designed to address the deficiencies of prior devices. Embodiments of the invention provide standalone interbody implants limiting or eliminating motion between the implant and the bone at the bone-to-implant interface. Movement between vertebra is instead located intra-implant, thereby allowing the implant to preserve motion of the spinal level while providing stability all without compromising the bone-implant interface. This allows the implant to be secured to the bone while the bone then grows into and/or onto the implant. Accordingly, embodiments of the invention address the deficiencies of the prior art.

Embodiments of the invention provides implants including one or more pieces that are designed to replace j oint motion from the implant-bone interface to an interface within the implant. Because the motion occurs within the implant instead of at the implant-bone interface, the implants relieve pain, reduce fibrous tissue formation, and alleviate pseudoarthrosis.

Implants according to embodiments of the invention can have tailored stiffnesses by modifying the geometry of the contact surfaces within the implant and or by modifying flexures of the implant. Modification of the flexures may involve modification of any one or more of width, height, curvature, or length of the flexures.

Primary fixation of the implant to the bone using screws, blades, or other fixation devices decreases motion at the bone-implant interface. The flexibility of the implant itself decreases motion between the bone and the implant at the bone-implant interface. Additionally, porous and/or roughened surfaces and/or volumes of the implant facilitate boney attachment of the bone to the implant, further reducing the likelihood of movement at the bone-implant interface.

In some embodiments, the use of three-dimensional (3D) printing to form the implant or portions thereof minimizes the number of parts needed to assemble the implant. Additionally, the use of 3D printing permits the creation of geometries that may not be available with traditional machining techniques. Some embodiments are formed as a single-piece implant. In other embodiments, the implant is manufactured in two or more pieces that are assembled using sliding or pressing assembly. The use of multiple-piece implants of some embodiments facilitates certain advantages such as decreasing the contact gap, improving the surface finish, machinability, or the like.

3D printing, while providing certain advantages, suffers from minimum distance requirements between objects or portions of the object, otherwise the features will fuse together. Traditional machining also has kerf limitations. Multi-piece manufacturing approaches allows for a desired performance of the implant while avoiding some of these limitations. Multi-piece construction also better allows for individualized control of other features such as surface finish, coatings, or variable-material compositions of the implant. One example of a variable-material composition is one material at the surface for promoting bone on-growth or ingrowth, and one material interior to the implant for intra-implant motion (e.g., rolling motion).

Flexure curvature and thickness can be altered in some embodiments in controlled ways by inserting contact surfaces. By way of example, an insert can be shaped to induce a specific stress in one or more flexures to alter kinematics that would not otherwise be possible. Additionally, implant inserts can be mixed and matched in some embodiments to create patient-specific implants.

According to embodiments of the invention, an interbody implant is configured to permit intra-implant movement while minimizing movement at a bone-implant interface, and includes an implant body formed to permit a desired range of intra-implant movement between an upper surface thereof and a lower surface thereof, an upper fixation element adapted to secure an upper portion of the implant body to a superior bone, and a lower fixation element adapted to secure a lower portion of the implant body to an inferior bone.

According to some embodiments, the implant body is formed as a single piece. According to some embodiments, the implant is formed as a plurality of pieces assembled together. According to some embodiments, one or more of the pieces assembled together includes a piece formed from 3D printing. According to some embodiments, one or more of the pieces assembled together includes a piece formed using a machining process. According to some embodiments, the upper fixation element includes a first bone screw passing through an upper aperture of the implant body, and wherein the lower fixation element includes a second bone screw passing through a lower aperture of the implant body.

According to some embodiments, each fixation element includes an element such as a screw or a blade. According to some embodiments, an upper surface of the implant body and a lower surface of the implant body include roughened or porous surfaces to facilitate bone on-growth or ingrowth. According to some embodiments, the upper portion of the implant body is flexibly affixed to the lower portion of the implant via a plurality of flexures extending between the upper portion and the lower portion.

According to some embodiments, the implant body includes the upper portion of the implant body, the lower portion of the implant body, a plurality of flexures extending between the upper portion of the implant body and the lower portion of the implant body, wherein the upper portion, the lower portion, and the plurality of flexures are formed as a unitary construct, an upper porous insert formed to promote bone ongrowth or ingrowth affixed to the upper portion, a lower porous insert formed to promote bone ongrowth or ingrowth affixed to the lower portion, and an insert inserted between the upper portion of the implant body and the lower portion of the implant body to modify a kinematic profile between the upper portion of the implant body and the lower portion of the implant body imparted by the plurality of flexures in the absence of the insert.

According to some embodiments, an interbody implant is configured to permit intra-implant movement while minimizing movement at a bone-implant interface and includes an implant body including a superior portion connected to an inferior portion by a plurality of flexures formed to permit a desired range of intra-implant movement between a superior surface thereof and an inferior surface thereof, a superior fixation element adapted to secure the superior portion of the implant body to a superior bone, and an inferior fixation element adapted to secure the inferior portion of the implant body to an inferior bone.

According to some embodiments, the implant is formed as a plurality of pieces assembled together using an assembly method selected from the group consisting of a sliding assembly method and a press-fit assembly method. According to some embodiments, one or more of the pieces assembled together includes a piece formed from three-dimensional (3D) printing. According to some embodiments, one or more of the pieces assembled together includes a piece formed using a machining process.

According to some embodiments, the superior fixation element includes a first bone screw passing through a superior aperture of the implant body, and wherein the inferior fixation element includes a second bone screw passing through an inferior aperture of the implant body. According to some embodiments, each fixation element includes an element selected from the group consisting of a screw and a blade.

According to some embodiments, a superior surface of the implant body and an inferior surface of the implant body include roughened or porous surfaces to facilitate bone on-growth or ingrowth. According to some embodiments, the superior portion of the implant body is flexibly affixed to the inferior portion of the implant via a plurality of flexures extending between the superior portion and the inferior portion.

According to some embodiments, the implant body includes the superior portion of the implant body, the inferior portion of the implant body, a plurality of flexures extending between the superior portion of the implant body and the inferior portion of the implant body, wherein the superior portion, the inferior portion, and the plurality of flexures are formed as a unitary construct, a superior porous insert formed to promote bone ongrowth or ingrowth affixed to the superior portion, an inferior porous insert formed to promote bone ongrowth or ingrowth affixed to the inferior portion, and an insert inserted between the superior portion of the implant body and the inferior portion of the implant body to modify a kinematic profile between the superior portion of the implant body and the inferior portion of the implant body imparted by the plurality of flexures in the absence of the insert.

According to some embodiments, the plurality of flexures are modifiable to modify a stiffness of the implant body via modifications of the flexures including a modification selected from the group consisting of a width of the flexure, a height of the flexure, a curvature of the flexure, a thickness of the flexure, and a length of the flexure.

Embodiments of the invention provide implants including one or more pieces that are designed to replace joint motion from the implant-bone interface to an interface within the implant. Because the motion occurs within the implant instead of at the implant-bone interface, the implants relieve pain, reduce fibrous tissue formation, and alleviate pseudoarthrosis.

Implants according to embodiments of the invention can have tailored stiffnesses by modifying the geometry of the contact surfaces within the implant and or by modifying flexures of the implant. Modification of the flexures may involve modification of any one or more of width, height, curvature, or length of the flexures.

Primary fixation of the implant to the bone using screws, blades, or other fixation devices decreases motion at the bone-implant interface. The flexibility of the implant itself decreases motion between the bone and the implant at the bone-implant interface. Additionally, porous and/or roughened surfaces and/or volumes of the implant facilitate boney attachment of the bone to the implant, further reducing the likelihood of movement at the bone-implant interface.

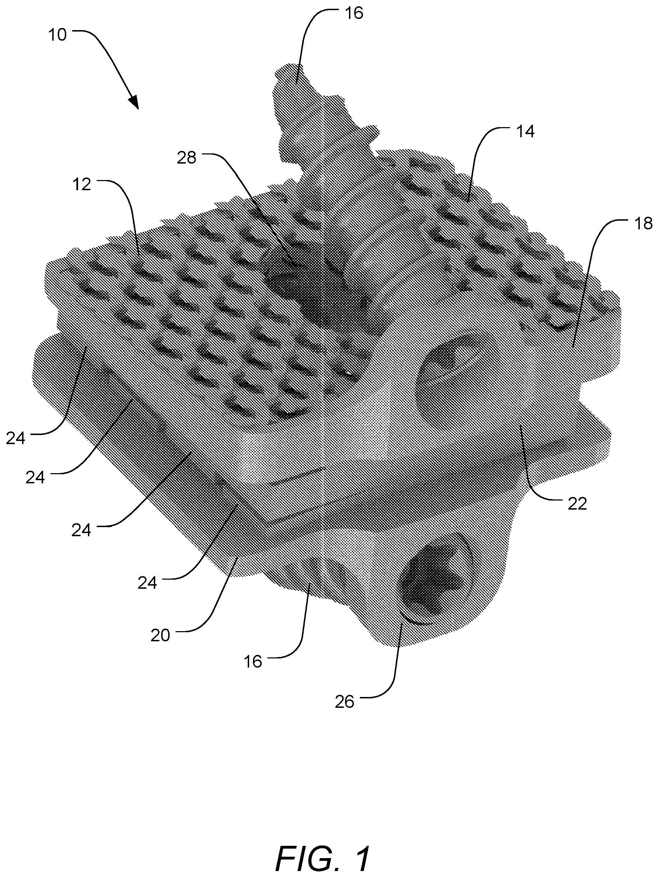

Figure 1 illustrates one embodiment of a standalone interbody implant 10 having an implant body 12 formed as a unitary construct. The implant body 12 of some embodiments is formed by a 3D printing method, which in some instances allows formation of a roughened or porous upper surface 14 and a corresponding porous lower surface (not shown in Figure 1) as disclosed in U.S. Patent Application Publication No. 2017/0156880A1. The roughened or porous surfaces facilitate bone ingrowth and/or ongrowth to the implant, whereby the bony growth more-securely fixes the implant 10 into the intervertebral space after implantation. The bony growth further secures the implant 10 against movement of the implant relative to the bone while initially the implant 10 is secured against such motion by one or more fixation elements 16 in each of the superior and inferior directions, depicted in Figure 1 as bone screws. Alternate fixation elements 16 include elements such as blades and the like. As depicted in Figure 1, the bone screws may be any type of bone screw, such as a hexalobe bone screw sized appropriately for the vertebral body into which the fixation element 16 is to be fastened.

The implant body 12 illustrated in Figure 1 includes an upper portion 18 (which may alternately be termed a superior portion as after implantation in the body it is oriented in a superior direction relative to the remainder of the implant body 12) and a lower portion 20 (which may alternately be termed an inferior portion due to its relative position after implantation). A central portion 22 is affixed to and extends between the upper portion 18 and the lower portion 20. In the illustrated embodiment, the upper portion 18 and the lower portion 20 are comparatively rigid (and may have a rigidity generally approximating the rigidity of cortical bone of the vertebral body) and the central portion 22 provides relative motion between the upper portion 18 and the lower portion 20. This relative motion may include, for example, medial-lateral rolling motion and anterior-posterior rolling motion.

In some embodiments, one axis of motion (e.g., medial-lateral rolling motion) is provided by an interface between the central portion 22 and either of the upper portion 18 or the lower portion 20. Another axis of motion (e.g., anterior-posterior rolling motion) is provided by an interface between the central portion 22 and the other of the upper portion 18 or the lower portion 20. The relative motion between portions of the body 12 is provided by one or more leaf springs or flexures 24 extending between the central portion 22 and the upper portion 18 and between the central portion 22 and the lower portion 20.

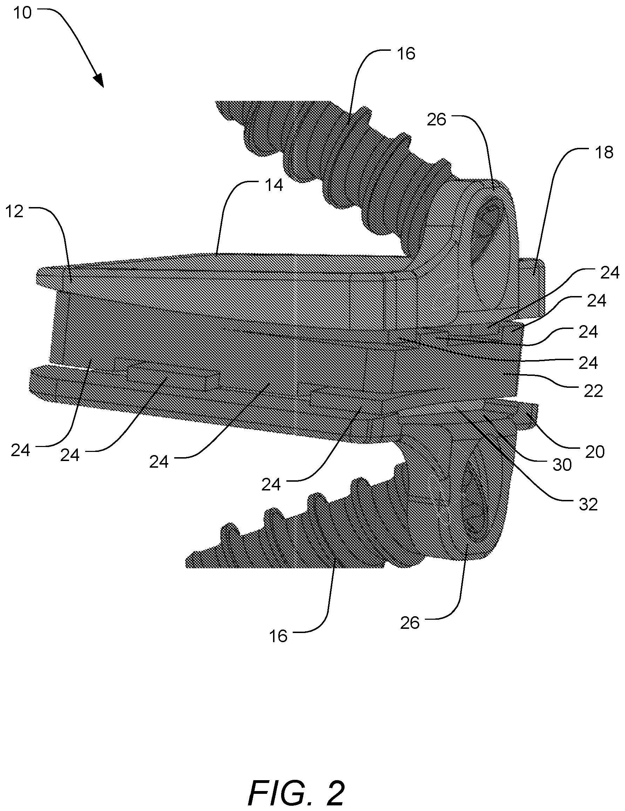

In the embodiment illustrated in Figure 1, the flexures 24 are formed of the same material as the rest of the implant body 12. One set of the flexures 24 in this embodiment extend in a generally medial-lateral direction (upon implantation) between the central portion 22 and the lower portion 20, thereby providing the medial-lateral rolling motion therebetween. Another set of the flexures 24 in this embodiment (not shown in Figure 1 but illustrated in the embodiment of Figure 2) extend in a generally anterior-posterior direction (upon implantation) between the central portion 22 and the upper portion 18, thereby providing the anterior-posterior rolling motion therebetween. Those of ordinary skill in the art will readily recognize that the orientation of the flexures between the upper and lower parts of the implant body 12 could be reversed while still providing two axes of rolling motion to the implant 10.

The fixation elements 16 pass through angled apertures 26 formed in the upper portion 18 and the lower portion 20. The angled aperture 26 of the upper portion 18 is angled upward such that the fixation element 16 can pass therethrough and enter a vertebral body above the implant 10. The angled aperture 26 of the lower portion 20 is angled downward such that the fixation element 16 can pass therethrough and enter a vertebral body below the implant 10. The implant body 12 of some embodiments includes one or more central cavities 28 that may be adapted to receive a bone graft mixture prior to or upon implantation of the implant 10 to promote bony growth into the implant, as is known in the art.

To implant the implant 10, the surgeon accesses the intervertebral space and prepares it as is known in the art (e.g., by removing and remaining disc material and potentially by preparing the surfaces of the vertebral bodies. The implant body 12 is inserted into the intervertebral space (after insertion of the bone graft material into the cavity 28 or cavities 28, if any) and is positioned as desired. Then, the implant body 12 is secured against movement as a first fixation element 16 is inserted through its angled aperture 26 into a vertebral body. The second fixation element 16 is then inserted through its angled aperture 26 into the other vertebral body.

In some embodiments, the implant body 12 may be formed in multiple elements that are then joined together. For example, in some embodiments, the upper portion 18, the central portion 22, and the lower portion 20 may be individually formed. Any of these portions may be formed with the flexures 24 and the flexures then affixed to the other portions to join them together. Alternatively, the flexures 24 may be separately formed and then affixed to extend between and join the portions together in their respective positions.

In some embodiments, including embodiments in which the implant body 12 is generally constructed as a single element and embodiments in which the implant body 12 is formed of multiple elements, the implant body 12 may be provided with one or more receptacles adapted to receive one or more inserts therein. By way of example, the embodiment of Figure 2 includes one receptacle 30 and one insert 32. The provision of receptacles such as receptacle 30 and of inserts such as insert 32 permit the characteristics of the implant 10, such as kinematics of rolling motion in one or more axes, to be modified as desired. In some embodiments, the material of the insert(s) may be similar to a material of the implant body 12 (e.g., titanium, a titanium alloy, tantalum, a tantalum alloy or any other biocompatible material), and in other embodiments, the material of the insert(s) may vary from the material of the implant body 12. The insert(s) 32 of some embodiments may be selected and inserted after manufacture of the implant body 12 as desired to achieve a desired implant performance.

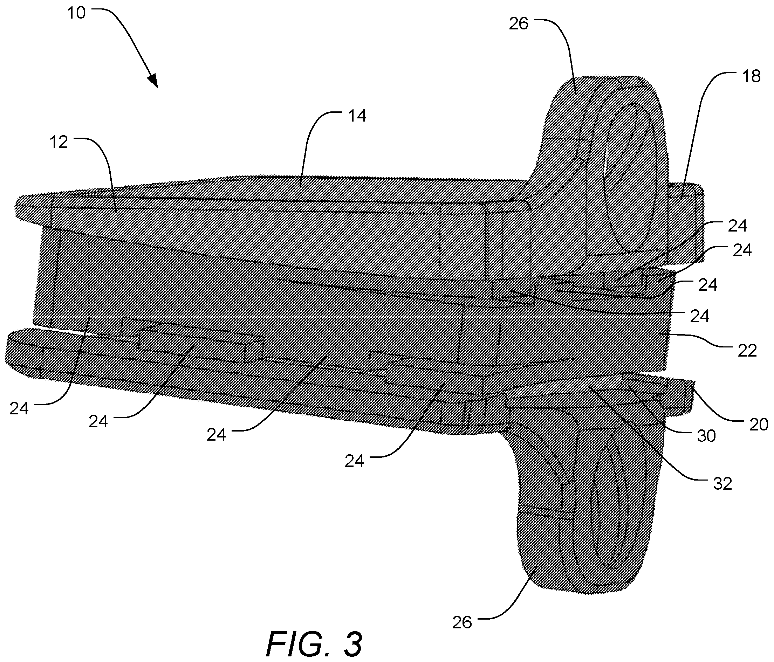

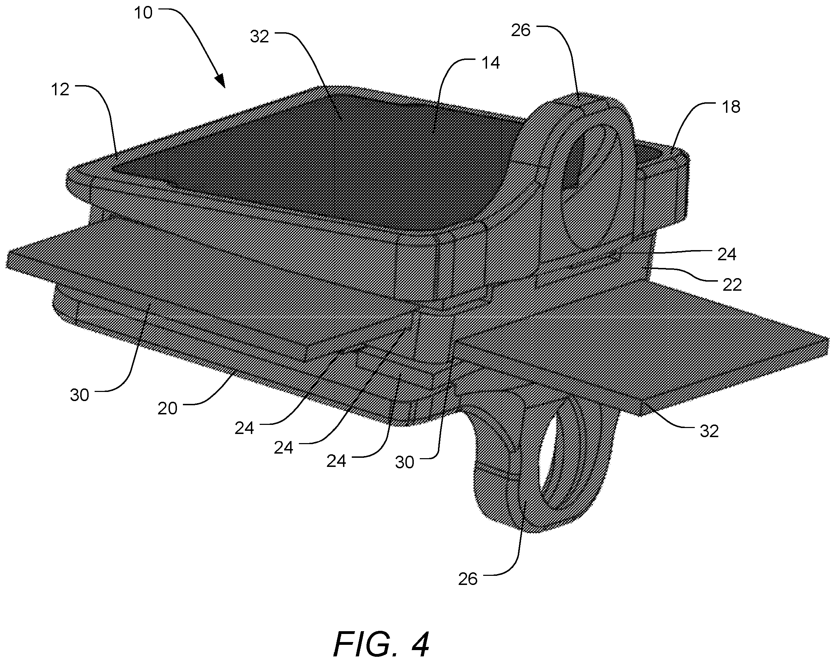

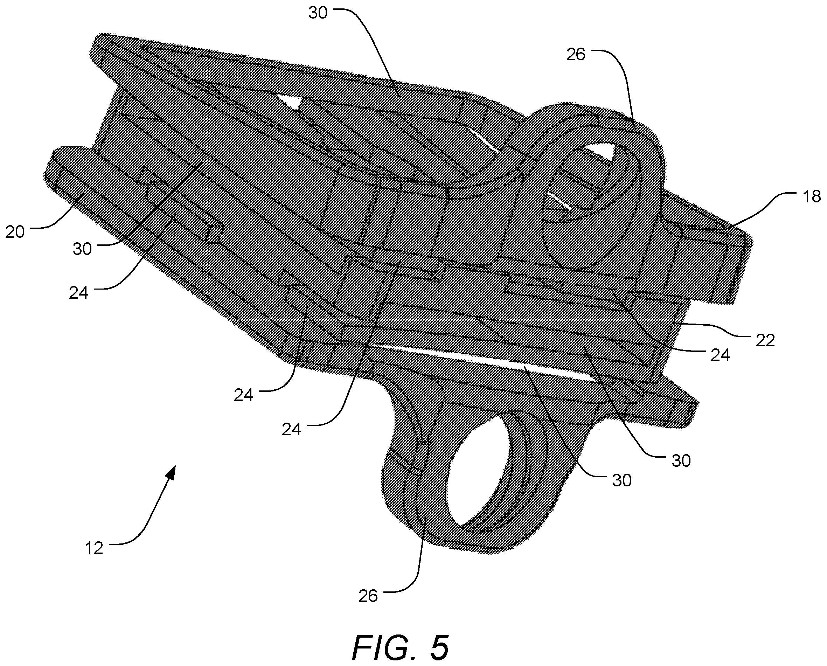

Figure 2 illustrates an embodiment with a single insert 32. Figure 3 illustrates another view of such embodiment in which the fixation elements 16 are omitted for clarity of illustration. Figure 4 illustrates an embodiment with up to four inserts 32. Two inserts 32 of this embodiment are inserted generally in receptacles 30 located in the central portion 22 and serve to modify characteristics associated with the rolling motion provided by the implant 10. To other inserts 32 of this embodiment are inserted into receptacles 30 located in the upper portion 18 and the lower portion 20 and serve to provide the roughened or porous upper surface 14 and lower surface of the implant 10. Figure 5 illustrates an embodiment of the implant body 12 with four receptacles 32 as in the illustration of Figure 4, but without the inserts 32 being depicted.

## Claims

1. An interbody implant (10) configured to permit intra-implant movement while minimizing movement at a bone-implant interface, comprising:
an implant body (12) formed to permit movement between an upper surface (14) thereof and a lower surface thereof;
an upper fixation element (16) adapted to secure an upper portion (18) of the implant body (10) to a superior bone; and
a lower fixation element (16) adapted to secure a lower portion (20) of the implant body (10) to an inferior bone,
**characterized in that** the interbody implant (10) further comprises:
a first flexure (24) comprising a first leaf spring disposed between a central portion (22) of the implant body (10) and the upper portion of the implant body (10); and
a second flexure (24) comprising a second leaf spring disposed between the central portion of the implant body (10) and the lower portion of the implant body (10).

2. The interbody implant (10) as recited in claim 1, wherein the first leaf spring, the second leaf spring, the central portion of the implant body (10), the upper portion of the implant body (10), and the lower portion of the implant body (10) are together formed as a single piece.

3. The interbody implant (10) as recited in claim 1, wherein each of the upper surface and the lower surface comprises at least one of a roughened surface and a porous surface configured to facilitate bony growth to more securely fix the interbody implant (10) into an intervertebral space.

4. The interbody implant (10) as recited in claim 3, wherein the upper surface, the lower surface, the first flexure, the second flexure, and the central portion of the implant body (10) are formed together via three-dimensional (3D) printing.

5. The interbody implant (10) as recited in claim 3, wherein the interbody implant (10) comprises a piece formed using a machining process.

6. The interbody implant (10) as recited in claim 1, wherein one of the first flexure and the second flexure is configured to provide medial-lateral rolling motion, and another of the first flexure and the second flexure is configured to provide anterior-posterior rolling motion.

7. The interbody implant (10) as recited in claim 1, wherein the implant body (10) further comprises a receptacle (30) configured to receive an insert (32).

8. The interbody implant (10) as recited in claim 7, wherein the insert (32) comprises at least one of titanium, a titanium alloy, tantalum, and a tantalum alloy.

9. The interbody implant (10) as recited in claim 7, wherein the insert (32) is configured to modify kinematics of rolling motion of the first flexure and the second flexure.

10. The interbody implant (10) as recited in claim 1, further comprising: a first insert configured to modify a rolling motion of the first flexure; a second insert configured to modify a rolling motion of the second flexure; a third insert configured to provide a roughened or porous upper surface; and a fourth insert configured to provide a roughened or porous lower surface.

11. The interbody implant (10) as recited in claim 1, wherein the first flexure and the second flexure are each modifiable to modify a stiffness of the implant body (10) via modifications of the first flexure and the second flexure comprising a modification selected from the group consisting of a width of the flexure, a height of the flexure, a curvature of the flexure, a thickness of the flexure, and a length of the flexure.

## Patentansprüche

1. Zwischenwirbelimplantat (10), das so konfiguriert ist, dass es Bewegungen innerhalb des Implantats zulässt, während es Bewegungen an einer Schnittstelle zwischen Knochen und Implantat minimiert, umfassend:
einen Implantatkörper (12), der so ausgebildet ist, dass er Bewegungen zwischen einer Oberseite (14) desselben und einer Unterseite desselben erlaubt;
ein oberes Fixierungselement (16), das dazu ausgelegt ist, einen oberen Abschnitt (18) des Implantatkörpers (10) an einem oberen Knochen zu befestigen; und
ein unteres Fixierungselement (16), das dazu ausgelegt ist, einen unteren Abschnitt (20) des Implantatkörpers (10) an einem unteren Knochen zu befestigen,
**dadurch gekennzeichnet, dass** das Zwischenwirbelimplantat (10) ferner Folgendes umfasst:
ein erstes Biegeelement (24) mit einer ersten Blattfeder, die zwischen einem mittleren Abschnitt (22) des Implantatkörpers (10) und dem oberen Abschnitt des Implantatkörpers (10) angeordnet ist; und
ein zweites Biegeelement (24) mit einer zweiten Blattfeder, die zwischen dem mittleren Abschnitt des Implantatkörpers (10) und dem unteren Abschnitt des Implantatkörpers (10) angeordnet ist.

2. Zwischenwirbelimplantat (10) nach Anspruch 1, wobei die erste Blattfeder, die zweite Blattfeder, der mittlere Abschnitt des Implantatkörpers (10), der obere Abschnitt des Implantatkörpers (10) und der untere Abschnitt des Implantatkörpers (10) zusammen als ein einziges Stück ausgebildet sind.

3. Zwischenwirbelimplantat (10) nach Anspruch 1, wobei die Oberseite und die Unterseite jeweils mindestens eines von einer angerauten Oberfläche und einer porösen Oberfläche umfasst, die dazu ausgebildet ist, das Knochenwachstum zu erleichtern, um das Zwischenwirbelimplantat (10) sicherer in einem Zwischenwirbelraum zu fixieren.

4. Zwischenwirbelimplantat (10) nach Anspruch 3, wobei die Oberseite, die Unterseite, das erste Biegeelement, das zweite Biegeelement und der mittlere Abschnitt des Implantatkörpers (10) gemeinsam durch dreidimensionalen Druck (3D-Druck) hergestellt sind.

5. Zwischenwirbelimplantat (10) nach Anspruch 3, wobei das Zwischenwirbelimplantat (10) ein unter Verwendung eines spanenden Verfahrens geformtes Teil umfasst.

6. Zwischenwirbelimplantat (10) nach Anspruch 1, wobei eines von dem ersten Biegeelement und dem zweiten Biegeelement dazu ausgebildet ist, eine mediallaterale Abrollbewegung zu ermöglichen, und ein anderes von dem ersten Biegeelement und dem zweiten Biegeelement dazu ausgebildet ist, eine anteriorposteriore Abrollbewegung zu ermöglichen.

7. Zwischenwirbelimplantat (10) nach Anspruch 1, wobei der Implantatkörper (10) ferner eine Aufnahme (30) umfasst, die dazu ausgebildet ist, einen Einsatz (32) aufzunehmen.

8. Zwischenwirbelimplantat (10) nach Anspruch 7, wobei der Einsatz (32) mindestens eines von Titan, einer Titanlegierung, Tantal und einer Tantallegierung umfasst.

9. Zwischenwirbelimplantat (10) nach Anspruch 7, wobei der Einsatz (32) dazu ausgebildet ist, die Kinematik der Abrollbewegung des ersten Biegeelements und des zweiten Biegeelements zu modifizieren.

10. Zwischenwirbelimplantat (10) nach Anspruch 1, das ferner Folgendes umfasst: einen ersten Einsatz, der dazu ausgebildet ist, eine Abrollbewegung des ersten Biegeelements zu modifizieren; einen zweiten Einsatz, der dazu ausgebildet ist, eine Abrollbewegung des zweiten Biegeelements zu modifizieren; einen dritten Einsatz, der dazu ausgebildet ist, eine angeraute oder poröse Oberseite bereitzustellen; und einen vierten Einsatz, der dazu ausgebildet ist, eine angeraute oder poröse Unterseite bereitzustellen.

11. Zwischenwirbelimplantat (10) nach Anspruch 1, wobei das erste Biegeelement und das zweite Biegeelement jeweils modifizierbar ist, um eine Steifigkeit des Implantatkörpers (10) über Modifikationen des ersten Biegeelements und des zweiten Biegeelements zu modifizieren, die eine Modifikation umfassen, die aus der aus einer Breite des Biegeelements, einer Höhe des Biegeelements, einer Krümmung des Biegeelements, einer Dicke des Biegeelements und einer Länge des Biegeelements bestehenden Gruppe ausgewählt ist.

## Revendications

1. Implant intersomatique (10) configuré pour permettre un mouvement intra-implantaire tout en minimisant le mouvement à une interface os-implant, comprenant:
un corps d'implant (12) formé pour permettre un mouvement entre une surface supérieure (14) de celui-ci et une surface inférieure de celui-ci;
un élément de fixation supérieur (16) adapté pour fixer une partie supérieure (18) du corps d'implant (10) à un os supérieur; et
un élément de fixation inférieur (16) adapté pour fixer une partie inférieure (20) du corps d'implant (10) à un os inférieur,
**caractérisé en ce que** l'implant intersomatique (10) comprend en outre:
un premier élément flexible (24) comprenant un premier ressort à lame disposé entre une partie centrale (22) du corps d'implant (10) et la partie supérieure du corps d'implant (10); et
un second élément flexible (24) comprenant un second ressort à lame disposé entre la partie centrale du corps d'implant (10) et la partie inférieure du corps d'implant (10).

2. Implant intersomatique (10) selon la revendication 1, dans lequel le premier ressort à lame, le second ressort à lame, la partie centrale du corps d'implant (10), la partie supérieure du corps d'implant (10) et la partie inférieure du corps d'implant (10) sont formés d'une seule pièce.

3. Implant intersomatique (10) selon la revendication 1, dans lequel chacune de la surface supérieure et de la surface inférieure comprend au moins l'une d'une surface rugueuse et d'une surface poreuse configurée pour favoriser la croissance osseuse pour fixer plus solidement l'implant intersomatique (10) dans un espace intersomatique.

4. Implant intersomatique (10) selon la revendication 3, dans lequel la surface supérieure, la surface inférieure, le premier élément flexible, le second élément flexible et la partie centrale du corps d'implant (10) sont formés ensemble par impression tridimensionnelle (3D).

5. Implant intersomatique (10) selon la revendication 3, dans lequel l'implant intersomatique (10) comprend une pièce formée au moyen d'un processus d'usinage.

6. Implant intersomatique (10) selon la revendication 1, dans lequel l'une du premier élément flexible et du second élément flexible est configuré pour assurer un mouvement de roulement médio-latéral, et un autre du premier élément flexible et du second élément flexible est configuré pour assurer un mouvement de roulement antéro-postérieur.

7. Implant intersomatique (10) selon la revendication 1, dans lequel le corps d'implant (10) comprend en outre un réceptacle (30) configuré pour recevoir un insert (32).

8. Implant intersomatique (10) selon la revendication 7, dans lequel l'insert (32) comprend au moins l'un parmi le titane, un alliage de titane, le tantale et un alliage de tantale.

9. Implant intersomatique (10) selon la revendication 7, dans lequel l'insert (32) est configuré pour modifier la cinématique du mouvement de roulement du premier élément flexible et du second élément flexible.

10. Implant intersomatique (10) selon la revendication 1, comprenant en outre: un premier insert configuré pour modifier un mouvement de roulement du premier élément flexible; un second insert configuré pour modifier un mouvement de roulement du second élément flexible; un troisième insert configuré pour présenter une surface supérieure rugueuse ou poreuse; et un quatrième insert configuré pour présenter une surface inférieure rugueuse ou poreuse.

11. Implant intersomatique (10) selon la revendication 1, dans lequel le premier élément flexible et le second élément flexible sont chacun modifiables pour modifier une rigidité du corps d'implant (10) par le biais de modifications du premier élément flexible et du second élément flexible comprenant une modification choisie dans le groupe consistant en une largeur de l'élément flexible, une hauteur de l'élément flexible, une courbure de l'élément flexible, une épaisseur de l'élément flexible et une longueur de l'élément flexible.
